# FASCICULE DE BREVET EUROPEEN

(11) **EP 0 832 644 B1**
(45) Date de publication et mention de la délivrance du brevet: **02.10.2002**
(21) Numéro de dépôt: 97401817.8
(22) Date de dépôt: 28.07.1997
(51) Int. Cl.: A61K 7/48

(54) **Utilisation d'un polydiméthysiloxane à groupements glucosides comme agent hydratant dans une composition cosmétique ou dermatologique**
Verwendung von einem Glykosid-Polydimethylsiloxan als Feuchthaltemittel in einer kosmetischen oder dermatologischen Zusammensetzung
Use of glycosidic polydimethylsiloxane as hydrating agent in a cosmetic or dermatological composition

(30) Priorité: 30.09.1996 FR 9611877
(43) Date de publication de la demande: 01.04.1998
(73) Titulaire: L'OREAL, 75008 Paris (FR)
(72) Inventeur: Afriat, Isabelle, 75014 Paris (FR); Gagnebien, Didier, Westfield 07090, New Jersey (US)
(74) Mandataire: Andral, Christophe André Louis

(56) Documents cités:
- US-A- 5 428 142
- DATABASE WPI Week 9516 Derwent Publications Ltd., London, GB; AN 95-118650 XP002031473 "Reduced oiliness external agent for skin and hair care - contg. organo-polysiloxane seriv. having sugar residue and polyhydric alcoho(s)" & JP 07 041 414 A (SHISEIDO) , 10 février 1995
- DATABASE WPI Week 9516 Derwent Publications Ltd., London, GB; AN 95-118954 XP002031474 "Cleaning agent for external application agent for skin, e.g. shampoos - is formed by blending organopolysiloxane deriv(s) having a sugar residue with anionic, ampholytic and/or nonionic surfactant" & JP 07 041 794 A (SHISEIDO) , 10 février 1995
- DATABASE WPI Week 9516 Derwent Publications Ltd., London, GB; AN 95-118651 XP002031475 "Emulsification compsn. for external application for anti-sun-tan contg. organopolysiloxane deriv. with sugar residue, polyoxyalkylene modified organopolysiloxane and oil component for skin and hair care" & JP 07 041 415 A (SHISEIDO) , 10 février 1995

## Description

L'invention se rapporte à l'utilisation, à titre d'agent hydratant, d'un polydiméthylsiloxane à groupements glucosides dans une composition cosmétique ou dermatologique, destinée au traitement de la peau humaine, y compris du cuir chevelu, et notamment à l'hydratation de la peau et au traitement des peaux sèches.

On sait qu'il est important, notamment pour éviter le vieillissement de la peau, de maintenir une bonne hydratation cutanée en vue de compenser la deshydratation que subit la peau exposée à l'atmosphère. C'est pourquoi les compositions cosmétiques et/ou dermatologiques contiennent des actifs appropriés destinés notamment à compenser cette deshydratation en augmentant la quantité d'eau présente au niveau des couches superficielles de l'épiderme.

Une telle amélioration de l'hydratation peut être réalisée en particulier à l'aide d'actifs apportant de l'eau, tels que les polyols et notamment la glycérine, les glycols et les sucres, ou bien à l'aide d'actifs protégeant le film hydro-lipidique de la peau en empêchant l'évaporation de l'eau par formation d'un effet barrière.

Les polyols ne peuvent être ajoutés en trop grande quantité dans une composition cosmétique ou dermatologique sous peine d'obtenir une composition collante, ce qui rend l'utilisation de cette dernière rédhibitoire pour l'utilisateur. En outre, la durée de l'hydratation apportée par les polyols est limitée dans le temps. Or, on cherche lors de l'application d'un produit cosmétique, une hydratation d'au moins une journée.

Les actifs renforçant l'effet barrière sont notamment des corps hydrocarbonés comme l'huile de vaseline. On observe généralement que l'effet désiré n'est pas obtenu immédiatement après application de la composition, mais progressivement et souvent seulement au bout de quelques heures.

Il subsiste donc le besoin d'actifs ayant de bonnes propriétés hydratantes, conférant une rémanence de l'hydratation et qui, en outre, ne confèrent pas un effet collant à la composition.

La demanderesse a maintenant trouvé qu'il était possible d'obtenir des compositions cosmétiques ou dermatologiques ayant rapidement d'excellentes propriétés hydratantes en y incorporant un polydiméthylsiloxane à groupements glucosides.

En outre, grâce à ce composé, si on souhaite que la composition contienne aussi un polyol, on peut introduire ce dernier en quantité plus faible et obtenir ainsi un produit moins collant que dans l'état de la technique.

Certes, il est connu d'utiliser des polysiloxanes à groupements glucosides dans des compositions cosmétiques. Ainsi, le document JP-A-7-41414 décrit l'utilisation de polysiloxanes porteurs de résidus glucidiques dans une composition topique hydratante contenant des polyols, ces polysiloxanes permettant d'inhiber le toucher gras induit par les polyols. Par ailleurs, le document US-A-5428142 décrit des silicones glucosidés ayant, notamment, des propriétés émollientes, utilisables comme tensioactifs très doux.

Toutefois, ces documents ne décrivent ni ne suggèrent nullement que ces composés puissent avoir eux-mêmes des propriétés hydratantes. Ces propriétés sont d'autant plus surprenantes que les polydiméthylsiloxanes sont connus pour être à la fois hydrophobes et perméables à la vapeur d'eau, ce qui signifie qu'ils ne peuvent ni apporter de l'eau à la peau ni produire d'effet barrière sur la peau, et qu'ils ne sont donc pas à priori appropriés pour améliorer l'hydratation de la peau. Par conséquent, il est tout à fait inattendu que des polydiméthylsiloxanes puissent avoir des propriétés hydratantes.

Ainsi, la présente invention a donc pour objet l'utilisation, comme actif hydratant dans une composition cosmétique pour le traitement de la peau, d'au moins un polydiméthylsiloxane à groupements glucosides de formule (I) : 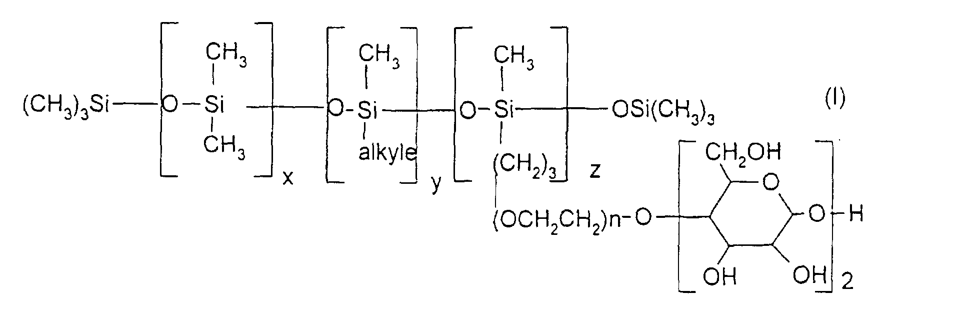 dans laquelle :
x, y et z représentent chacun un nombre entier positif allant de 1 à 10000, de préférence de 1 à 5000, et mieux de 1 à 2000,
n représente un nombre entier positif allant de 1 à 20, de préférence de 1 à 15,
le radical alkyle représente un radical hydrocarboné saturé, linéaire ou ramifié, comportant de 2 à 20, et de préférence de 2 à 8 atomes de carbone.

Dans la formule (I), le groupement : 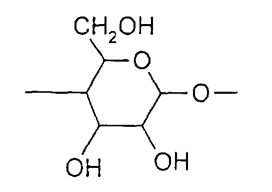 représente le groupement glucoside.

L'invention a également pour objet l'utilisation comme actif hydratant pour la préparation d'une composition dermatologique destinée au traitement de la peau, d'au moins un polydiméthylsiloxane à groupements glucosides de formule (I) indiquée ci-dessus.

L'invention a encore pour objet l'utilisation d'au moins un polydiméthylsiloxane à groupements glucosides de formule (I) indiquée ci-dessus dans une composition cosmétique pour obtenir une hydratation rémanente de la peau.

La présente invention a aussi pour objet l'utilisation d'au moins un polydiméthylsiloxane à groupements glucosides de formule (I) indiquée ci-dessus pour préparer une pommade ou un onguent dermatologique destiné au traitement thérapeutique de la peau sèche.

L'invention a en outre pour objet un procédé cosmétique pour hydrater la peau consistant à appliquer sur la peau une composition cosmétique contenant un polydiméthylsiloxane à groupements glucosides de formule (I) indiquée ci-dessus.

Les polydiméthylsiloxanes à groupements glucosides de formule (I) utilisables selon l'invention sont notamment ceux cités dans le document EP-A-612759 où ils sont décrits uniquement comme émulsionnants non-ioniques, peu irritants du fait de leur excellente biodégradabilité.

Dans les composés de formule (I) indiquée ci-dessus, le radical alkyle linéaire ou ramifié peut comporter de 2 à 20 et de préférence de 2 à 8 atomes de carbone. Ces radicaux peuvent être notamment choisis parmi les radicaux méthyle, éthyle, n-propyle, iso-propyle, n-butyle, iso-butyle, tert-butyle, n-pentyle, iso-pentyle, néopentyle, tert-pentyle, les radicaux hexyle comme le radical n-hexyle, les radicaux heptyle comme le radical n-heptyle, les radicaux octyle comme le radical n-octyle et iso-octyle.

Le polydiméthylsiloxane à groupements glucosides utilisé selon l'invention peut par exemple être présent dans la composition en une concentration allant de 1,5 à 9 % et de préférence de 1,8 à 6 % du poids total de la composition. Le polydiméthylsiloxane à groupements glucosides peut être introduit tel quel dans un milieu cosmétiquement ou dermatologiquement acceptable, ou bien, le plus souvent, être introduit dans ce milieu sous forme d'une solution, notamment dans une huile de silicone volatile. Les quantités indiquées ci-dessus sont les quantités en matière active (M.A.).

Comme polydiméthylsiloxane à groupements glucosides, on utilise, selon un mode préféré de réalisation de l'invention, un composé de formule (I) où le radical alkyle est un radical n-octyle. Ce composé est vendu sous la dénomination « SPG 128 VP » par la société Wacker ; il se présente sous forme d'une solution liquide à 15 % en matière active dans une cyclométhicone. On peut utiliser cette solution en une concentration allant de 10 à 60 %, et de préférence de 12 à 40 % du poids total de la composition.

La composition contenant le dérivé de formule (I) selon l'invention se présente de préférence sous forme d'une émulsion eau-dans-huile ayant l'aspect d'une crème ou d'un lait, éventuellement apte à mousser lorsqu'elle est mise sous pression (aérosol), ou encore sous forme de dispersions vésiculaires contenant des lipides ioniques et/ou non ioniques. Ces formes galéniques sont préparées selon les méthodes usuelles. Selon une forme préférée de réalisation, la composition est une émulsion fluide ayant une viscosité allant de 3 à 10 poises. Toutefois, d'autres formes galéniques comme des gels peuvent être envisagées.

Les quantités des différents constituants de la composition sont celles classiquement utilisées dans les domaines cosmétique et dermatologique.

Dans la composition, la proportion totale de corps gras peut aller de 0,1 à 50 %, et de préférence de 10 à 20 % du poids total de la composition. Les corps gras utilisés dans la composition sont choisis parmi ceux classiquement utilisés dans le domaine cosmétique ou dermatologique.

Comme corps gras utilisables dans l'invention, on peut par exemple citer les huiles d'origine minérale (huile de vaseline), les huiles d'origine végétale (huile d'abricot) et leurs dérivés hydrogénés, les huiles d'origine animale, les huiles de synthèse (polyisobutène hydrogéné), les huiles siliconées (cyclométhicone) et les huiles fluorées. Comme autres corps gras, on peut encore citer les alcools gras, les acides gras et les cires.

La composition peut contenir aussi un ou plusieurs co-émulsionnants, et notamment un co-émulsionnant polyglycérolé ayant un HLB (Hydrophilic Lipohilic Balance) allant de 4 à 7. Comme co-émulsionnant polyglycérolé, on peut citer par exemple les isostéarates de polyglycéryle et plus particulièrement l'isostéarate de polyglycéryle-4 vendu sous la dénomination « Isolan GI34 » par la société Goldschmidt.

Les co-émulsionnants peuvent être présents, dans la composition, en une proportion allant de 0,3 à 3 %, et de préférence de 0,5 à 1,5 % du poids total de l'émulsion.

De façon connue, la composition cosmétique ou dermatologique peut contenir également des adjuvants habituels dans le domaine cosmétique ou dermatologique, tels que les gélifiants hydrophiles ou lipophiles, les conservateurs, les antioxydants, les solvants, les parfums, les charges et les matières colorantes. Elle peut contenir aussi des actifs hydrophiles ou lipophiles et des filtres organiques ou minéraux tels que les pigments et notamment l'oxyde de titane. Les quantités de ces différents adjuvants et actifs sont celles classiquement utilisées dans les domaines considérés, et par exemple de 0,01 à 20 % du poids total de la composition. Ces adjuvants et actifs, selon leur nature, peuvent être introduits dans la phase grasse, dans la phase aqueuse et/ou dans des vésicules lipidiques.

Cette composition peut constituer notamment une composition de nettoyage, de protection, de traitement ou de soin pour le visage, pour le cou, pour les mains ou pour le corps, (par exemple crèmes de jour, crèmes de nuit, crèmes démaquillantes, crèmes ou huiles solaires, huiles pour le corps ou le visage, laits de nettoyage, laits de démaquillage, des laits corporels), une composition de maquillage (par exemple fond de teint), une composition de bronzage artificiel ou une composition pour le bain.

Les exemples qui suivent sont donnés à titre illustratif afin de mieux faire comprendre l'invention. Les quantités indiquées sont des pourcentages en poids.

Dans tous les exemples, les compositions sont préparées en versant la phase huileuse dans la phase aqueuse sous agitation.

### EXEMPLE 1 : FLUIDE HYDRATANT

### Phase huileuse :

- SPG 128 VP (à 15 % de M.A.) 20 %
- Polyisobutène hydrogéné 15 %

### Phase aqueuse :

- Glycérine 5 %
- Sulfate de magnésium (stabilisant) 0,7 %
- Eau qsp 100 %

On obtient un fluide ayant une viscosité de 3 poises, apte à hydrater la peau.

### EXEMPLE 2 : EMULSION DEMAQUILLANTE

### Phase huileuse :

- SPG 128 VP (à 15 % de M.A.) 15 %
- Huile de silicone volatile 9 %
- Huile de vaseline 6 %

### Phase aqueuse :

- Glycérine 5 %
- Sulfate de magnésium (stabilisant) 0,7 %
- Eau qsp 100 %

On obtient une émulsion hydratante fluide apte à démaquiller la peau tout en l'hydratant pendant au moins 24 heures.

### EXEMPLE 3 : EMULSION HYDRATANTE POUR LA PROTECTION SOLAIRE

### Phase huileuse :

- SPG 128 VP (à 15 % de M.A.) 20 %
- Huile de silicone volatile 5 %
- Polyisobutène hydrogéné 5 %
- Oxyde de titane 5 %

### Phase aqueuse :

- Glycérine 5 %
- Sulfate de magnésium (stabilisant) 0,7 %
- Eau qsp 100 %

On obtient une émulsion fluide apte à protéger la peau des effets du soleil tout en lui apportant une hydratation ayant une bonne rémanence pendant au moins 24 heures.

### EXEMPLE 4: FLUIDE HYDRATANT

### Phase huileuse :

- SPG 128 VP (à 15 % de M.A.) 20 %
- Polyisobutène hydrogéné 15 %
- Isostéarate de polyglycéryle-4 1 %

### Phase aqueuse :

- Glycérine 5 %
- Sulfate de magnésium (stabilisant) 0,7 %
- Eau qsp 100 %

On obtient un fluide apte à hydrater la peau pendant plusieurs heures.

### Test d'hydratation :

A l'aide d'un cornéomètre, on a réalisé un test de mesure de l'hydratation *in vivo* sur 16 sujets ayant la peau sèche. Le cornéomètre est un instrument couramment utilisé pour mesurer l'hydratation de la couche cornée de la peau. Cet instrument comporte un condensateur dont la capacité varie en fonction de la teneur en eau de la couche cornée.

Pour réaliser le test, on a appliqué 2 mg/cm² de produit sur la face interne de l'avant-bras de chaque sujet et on a mesuré l'hydratation au cornéomètre 1 heure et 24 heures après l'application.

On a testé comparativement une composition selon l'invention contenant 3 % en matière active de SPG 128 VP et 5 % de glycérol (composition A) et une composition de l'art antérieur comportant 3 % d'ester de sucre (mélange de dioléate de méthylglucose, d'huile de ricin hydrogénée et de cire d'abeille, vendu sous la dénomination Grillocose 481 par la société Grillo) et 5 % de glycérol (composition B).

Pour les 16 sujets, on a déterminé la différence entre l'hydratation mesurée une heure après application des compositions et l'hydratation mesurée 24 heures après application et on a fait la moyenne des résultats obtenus :
- pour la composition A selon l'invention, cette moyenne est de 7,375 ;
- pour la composition B, la moyenne est de 10,9375.

La décroissance de l'hydratation est donc plus forte avec la composition B. Un test de t apparié a montré que le résultat était significatif à 0,7 %.

Ainsi, ce test montre que le polydiméthylsiloxane à groupements glucosides selon l'invention hydrate la peau, et ce plus longtemps que l'ester de sucre de l'état de la technique.

## Revendications

1. Utilisation comme actif hydratant dans une composition cosmétique pour le traitement de la peau, d'au moins un polydiméthylsiloxane à groupements glucosides de formule (I) : 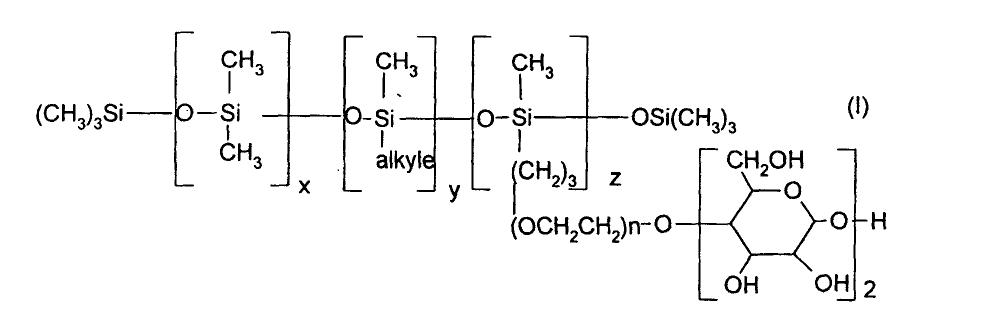 dans laquelle :
x, y et z représentent chacun un nombre entier positif allant de 1 à 10000,
n représente un nombre entier positif allant de 1 à 20,
le radical alkyle représente un radical hydrocarboné saturé, linéaire ou ramifié, comportant de 2 à 20 atomes de carbone,
la dite composition étant sous forme d'émulsion eau-dans-huite, comprenant au moins un polyol et contenant en outre au moins un co-émulsionnant choisi parmi les isostéarates de polyglycéryle.

2. Utilisation selon la revendication 1, **caractérisée en ce que** x, y et z représentent chacun un nombre entier positif allant de 1 à 5000.

3. Utilisation selon la revendication 1 ou 2, **caractérisée en ce que** n représente un nombre entier positif allant de 1 à 15.

4. Utilisation selon l'une quelconque des revendications précédentes, **caractérisée en ce que** le radical alkyle représente un radical hydrocarboné saturé comportant de 2 à 8 atomes de carbone.

5. Utilisation selon l'une quelconque des revendications précédentes, **caractérisée en ce que** le radical alkyle est choisi parmi les radicaux éthyle, propyle, butyle, pentyle, hexyle, heptyle, octyle.

6. Utilisation selon l'une quelconque des revendications précédentes, **caractérisée en ce que** la quantité de polydiméthylsiloxane à groupements glucosides dans la composition va, en matière active, de 1,5 à 9 % du poids total de la composition.

7. Utilisation selon l'une quelconque des revendications précédentes, **caractérisée en ce qu'**elle est fluide.

8. Utilisation selon l'une quelconque des revendications précédentes, **caractérisée en ce que** le polyol est la glycérine.

9. Procédé cosmétique pour hydrater la peau consistant à appliquer sur celle-ci une composition cosmétique comprenant au moins un polydiméthytsiloxane à groupements glucosides de formule (I) : 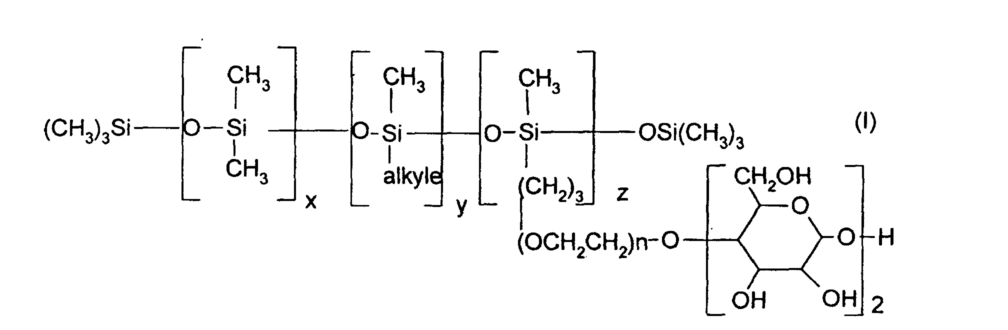 dans laquelle :
x, y et z représentent chacun un nombre entier positif allant de 1 à 10000,
n représente un nombre entier positif allant de 1 à 20,
le radical alkyle représente un radical hydrocarboné saturé, linéaire ou ramifié, comportant de 2 à 20 atomes de carbone,
la dite composition étant sous forme d'émulsion eau-dans-huile, comprenant au moins un polyol et contenant en outre au moins au moins un co-émulsionnant choisi parmi les isostéarates de polyglycéryle.

## Patentansprüche

1. Verwendung mindestens eines Polydimethylsiloxans mit Glucosidgruppen der Formel (I) als hydratisierenden Wirkstoff in einer kosmetischen Zusammensetzung zur Behandlung der Haut: 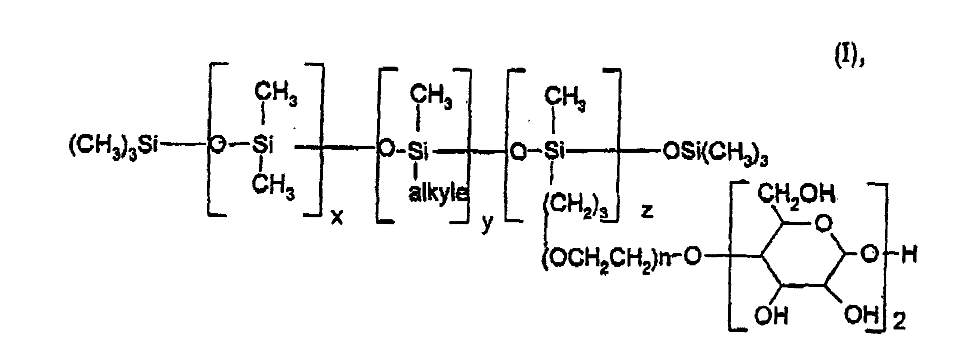 worin bedeuten:
x, y und z jeweils eine positive ganze Zahl im Bereich von 1 bis 10 000,
n eine positive ganze Zahl von 1 bis 20,
die Alkylgruppe eine gesättigte, geradkettige oder verzweigte Kohlenwasserstoffgruppe mit 2 bis 20 Kohlenstoffatomen,
wobei die Zusammensetzung in Form einer Wasser-in-Öl-Emulsion vorliegt, die mindestens ein Polyol und außerdem mindestens einen Coemulgator enthält, der unter den Polyglycerylisostearaten ausgewählt ist.

2. Verwendung nach Anspruch 1, **dadurch gekennzeichnet, daß** x, y und z jeweils eine positive ganze Zahl im Bereich von 1 bis 5000 bedeuten.

3. Verwendung nach Anspruch 1 oder 2, **dadurch gekennzeichnet, daß** n eine positive ganze Zahl im Bereich von 1 bis 15 bedeutet.

4. Verwendung nach einem der vorhergehenden Ansprüche, **dadurch gekennzeichnet, daß** die Alkylgruppe eine gesättigte Kohlenwasserstoffgruppe mit 2 bis 8 Kohlenstoffatomen ist.

5. Verwendung nach einem der vorhergehenden Ansprüche, **dadurch gekennzeichnet, daß** die Alkylgruppe unter den Gruppen Ethyl, Propyl, Butyl, Pentyl, Hexyl, Heptyl und Octyl ausgewählt ist.

6. Verwendung nach einem der vorhergehenden Ansprüche, **dadurch gekennzeichnet, daß** der als Wirkstoff ausgedrückte Mengenanteil des Polydimethylsiloxans mit Glucosidgruppen in der Zusammensetzung im Bereich von 1,5 bis 9 % des Gesamtgewichts der Zusammensetzung liegt.

7. Verwendung nach einem der vorhergehenden Ansprüche, **dadurch gekennzeichnet, daß** sie fluid ist.

8. Verwendung nach einem der vorhergehenden Ansprüche, **dadurch gekennzeichnet, daß** das Polyol das Glycerin ist.

9. Kosmetisches Verfahren zur Hydratisierung der Haut, das darin besteht, auf die Haut eine kosmetische Zusammensetzung aufzutragen, die mindestens ein Polydimethylsiloxan mit Glucosidgruppen der Formel (I) enthält: 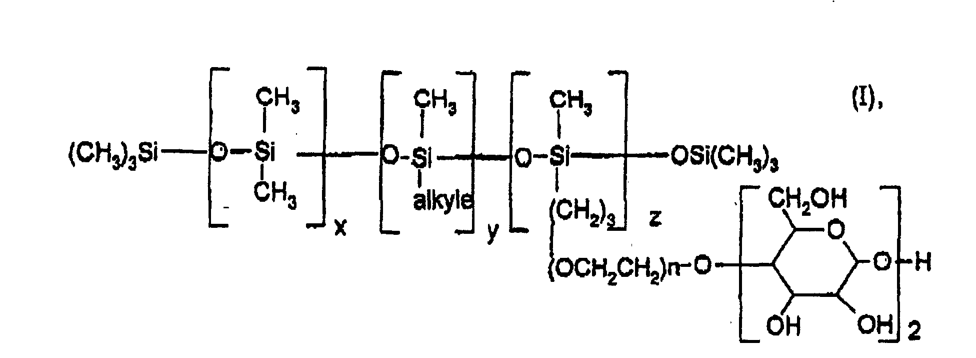 worin bedeuten:
x, y und z jeweils eine positive ganze Zahl im Bereich von 1 bis 10 000,
n eine positive ganze Zahl von 1 bis 20,
die Alkylgruppe eine gesättigte, geradkettige oder verzweigte Kohlenwasserstoffgruppe mit 2 bis 20 Kohlenstoffatomen,
wobei die Zusammensetzung in Form einer Wasser-in-Öl-Emulsion vorliegt, die mindestens ein Polyol und außerdem mindestens einen Coemulgator enthält, der unter den Polyglycerylisostearaten ausgewählt ist.

## Claims

1. Use, as moisturizing active agent in a cosmetic composition for treating the skin, of at least one polydimethylsiloxane containing glucoside groups, of formula (I): 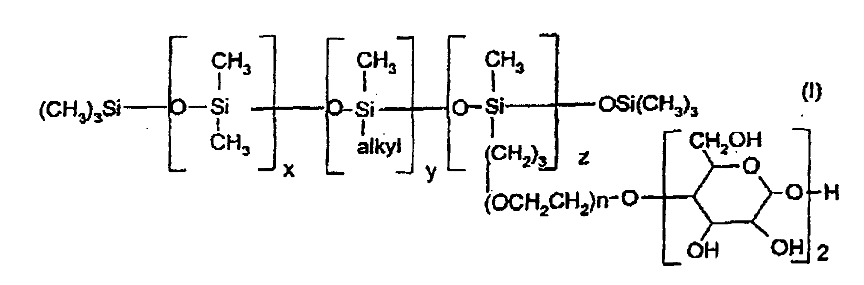 in which:
x, y and z each represent a positive integer ranging from 1 to 10,000,
n represents a positive integer ranging from 1 to 20,
the alkyl radical represents a linear or branched saturated hydrocarbon radical containing from 2 to 20 carbon atoms,
the said composition being in the form of a water-in-oil emulsion, comprising at least one polyol and further containing at least one co-emulsifier chosen from polyglyceryl isostearates.

2. Use according to claim 1, **characterized in that** x, y and z each represent a positive integer ranging from 1 to 5000.

3. Use according to Claim 1 or 2, **characterized in that** n represents a positive integer ranging from 1 to 15.

4. Use according to any one of the preceding claims, **characterized in that** the alkyl radical represents a saturated hydrocarbon radical containing from 2 to 8 carbon atoms.

5. Use according to any one of the preceding claims, **characterized in that** the alkyl radical is chosen from the ethyl, propyl, butyl, pentyl, hexyl, heptyl and octyl radicals.

6. Use according to any one of the preceding claims, **characterized in that** the amount of polydimethylsiloxane containing glucoside groups in the composition ranges, in active material terms, from 1.5 to 9% of the total weight of the composition.

7. Use according to any one of the preceding claims, **characterized in that** the emulsion is fluid.

8. Use according to any one of the preceding claims, **characterized in that** the polyol is glycerol.

9. Cosmetic process for moisturizing the skin, which consists in applying to the skin a cosmetic composition comprising at least one polydimethylsiloxane containing glucoside groups, of formula (I): 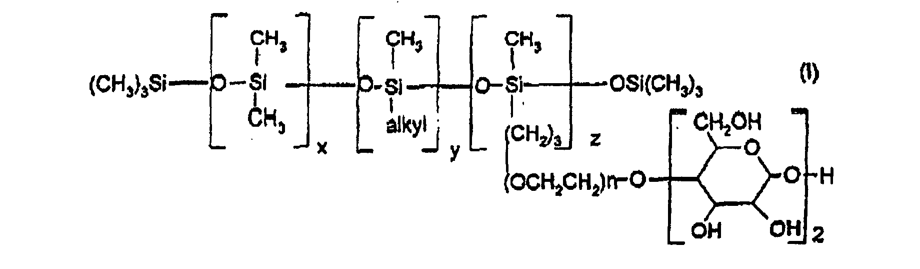 in which:
x, y and z each represent a positive integer ranging from 1 to 10,000,
n represents a positive integer ranging from 1 to 20,
the alkyl radical represents a linear or branched saturated hydrocarbon radical containing from 2 to 20 carbon atoms,
the said composition being in the form of a water-in-oil emulsion, comprising at least one polyol and further containing at least one co-emulsifier chosen from polyglyceryl isostearates.
